# EUROPEAN PATENT SPECIFICATION

(11) **EP 0 370 573 B1**
(45) Date of publication and mention of the grant of the patent: **23.08.1995**
(21) Application number: 89202922.4
(22) Date of filing: 20.11.1989
(51) Int. Cl.: A61K 49/00, A61K 39/21

(54) **Skin test and test kit for AIDS**
Hauttest und Testkit für AIDS
Test cutané et kit pour le dépistage du SIDA

(30) Priority: 21.11.1988 US 274197
(43) Date of publication of application: 30.05.1990
(73) Proprietor: Microgenesys, Inc., West Haven New Haven Connecticut 06516 (US)
(72) Inventor: Volvovitz, Franklin, New Haven, CT 06511 (US)
(74) Representative: Smulders, Theodorus A.H.J., Ir.

(56) References cited:
- EP-A- 265 785
- EP-A- 272 858
- EP-A- 0 174 805
- PROCEEDINGS OF THE NATL. ACADEMY OF SCIENCES USA, vol. 84; pp. 6924-6928/
- VACCINE, vol. 5, no. 2; pp. 90-101/
- NATURE, vol. 332, 21 August 1988; D. ZAGURY et al., pp. 728-731/
- BIOTECHNOLOGY, vol. 6, no. 3, March 1988, New York, NY (US); J. VAN BRUNT, pp. 259-264/
- A. GOODMAN-GILMAN et al., "The Pharmacological Basis of Therapeutics", 7th ed., 1985, MacMillan Publishing Co., New York, NY (US); p. 962/
- DATABASE WPI (L), Derwent; acc. no. 8212018E/

## Description

### BACKGROUND OF THE INVENTION

Human Immunodeficiency Virus Type-1 (HIV-1) is a retrovirus which causes systemic infection with a major pathology in the immune system. Clinical isolates of HIV-1 are also known as LAV (lymphadenopathy-associated virus), HTLV-III (human T-cell lymphotropic virus type III) or ARV (AIDS-associated retrovirus). HIV-1 infection causes Acquired Immune Deficiency Syndrome (AIDS). There is no specific treatment for this disease. This disease to date has been universally fatal and there have been no documented remissions.

Persons infected with HIV-1 show a progressive loss of immune function due primarily to the depletion of T4 lymphocytes. T4 lymphocytes are functionally defined as helper/inducer cells and serve an essential role in the recognition of antigens and the regulation of subsequent humoral and cellular immune responses. Persons with advance AIDS can become completely anergic including loss of the ability to respond to potent mitogens.

T4 lymphocytes are also called CD4 cells due to the presence on their surface of the CD4 molecule. This molecule is believed to be the receptor for HIV-1 providing the mechanism for entry into T4 lymphocytes. Nerve cells with this molecule on their surface are also infected by HIV-1.

The incubation period for AIDS is extremely long; many years may elapse following HIV-1 infection and the onset of clinical symptoms. The typical time between exposure to the virus and seroconversion has been estimated to be six to eight weeks, but this period is highly variable and some individuals were reported to have remained seronegative for many months following HIV-1 infection.

The clinical manifestations of HIV-1 infection include opportunistic infections, various malignancies and neurologic complications. As a group, opportunistic infections are the most common presenting clinical manifestations that ultimately lead to a diagnosis of AIDS and the most common cause of death in AIDS patients. The most important of these infections include Pneumocystis carinii pneumonia; toxoplasmosis; tuberculosis; viral infections due to herpes simplex, herpes zoster and cytomegalovirus; cryptococcal disease (especially meningitis); oral and esophageal candidiasis and cryptosporidiosis.

EP-A-0272858 discloses synthesis of HIV envelope protein gp160 in insect cells infected with recombinant baculoviruses. The gp160 protein is suggested for use in vaccines and immunometric assay reagents.

EP-A-0265785 discloses synthesis of HIV envelope proteins in recombinant baculovirus-infected insect cells. The envelope proteins are suggested for use in vaccines and immunodiagnostic assays.

Barr *et al*., Vaccine (1987) 5: 90-101, discloses expression of HIV envelope protein domains in recombinant yeast cells. The proteins were used as immunogens in experimental animals by perilymphnodal injection into rabbits. The HIV proteins stimulated antibody formation.

It is an object of this invention to provide a simple test which would be useful in determining whether or not one has an HIV-1 or AIDS infection.

It is another object of this invention to provide an inexpensive skin test which can be usefully employed to determine whether a human has an AIDS infection and wherein results of such test are relatively quickly available.

It is yet another object of this invention to provide a human test for determining whether or not a human has an AIDS infection, which test has relatively little, if any, side effects.

How these and other objects of this invention are achieved will become apparent in the light of the accompanying disclosure.

### SUMMARY OF THE INVENTION

The invention provides a use of an immunogenic HIV envelope protein free of antigenic human proteins for preparing a sterile preservative-free aqueous solution for intradermal administration to a human to test for a delayed type hypersensitivity reaction to HIV antigen.

Furthermore, the invention provides a use of an immunogenic HIV envelope protein free of antigenic human proteins for preparing a test kit for intradermal administration for assessment of HIV specific cellular immunologic response in humans, said test kit comprising a sealed container containing said HIV envelope protein in a preservative-free aqueous solution, and at least one sterile disposable syringe and needle.

Immunogenic AIDS virus envelope protein materials are employed in the practices of this invention to determine whether a human suffers from an AIDS infection by intradermally administering said material into the skin of a human and observing the site of administration or injection for an immune response, such as characteristic redness or swelling, at the site of administration or infection. The observation of an immune response at the site of administration or injection is indicative that the human suffers from an HIV-1 or AIDS infection.

It is preferred in the practices of this invention that the immunogenic AIDS virus envelope protein material be derived from synthetic or genetically engineered HIV-1 envelope protein produced in insect cells. A baculovirus expression vector containing a portion of HI V-1 envelope gene, env, is usefully employed to program cultured insect cells to produce the synthetic HIV-1 envelope protein. The synthetic gp160 antigen (HIV-1 gp160) is a glycoprotein with a molecular weight of approximately 160,000. The preparation of genetically engineered, recombinant immunogenic AIDS virus envelope protein material is disclosed in copending, coassigned U.S. patent application Serial No. 920,197 filed October 16, 1986 and in the corresponding EPO patent application published May 4, 1988, EP Patent Publication No. 0 265785.

The HIV envelope protein is harvested and purified from cultures of Lepidopteran (insect cells) following exposure to the recombinant baculovirus vector. The protein is then purified so that the resulting purified protein product or preparation contains not more than 5% insect and baculovirus proteins and is free of association with human blood or blood products.

Immunogenic AIDS or HIV-1 virus envelope protein material recovered from human T-cells infected with HIV-1 virus is also useful, but at present not preferred, in the practices of this invention. For the production of tise immunogenic material from T-cells infected with HIV-1, see U.S. Patent 4,725,609 granted February 16, 1988.

The above-mentioned immunogenic AIDS virus envelope protein materials are usefully employed in the practices of this invention in a delayed-type hypersensitivity (DTH) skin test or the determination of whether or not one to whom the test is administered has an AIDS or HIV-1 infection.

DTH skin testing has been used in assessing the cellular arm of the immune system in persons with suspected immunodeficiency due to infectious diseases and malignancies. DTH skin testing has also been used to predict morbidity in cancer and surgical patients. Heretofore, however, the use of immunogenic virus envelope protein material has not been used in DTH skin testing for AIDS infections. DTH skin testing employing immunogenic AIDS virus envelope protein material in accordance with this invention is employed as an aid in the assessment of HIV-1 specific cellular immunologic responses and the intradermal administration of this material in AIDS or HIV-1 infected persons should produce a characteristic DTH response (localized redness and induration) due to the release of specific lymphokines and mononuclear cell infiltration acting at the injection site. Although the administration of the skin test in accordance with this invention is not intended to be an immunizing dose, the administration of the immunogenic AIDS virus envelope protein material in accordance with this invention, particularly the gp160 protein, may seroconvert for all AIDS or HIV-1 envelope antigens.

The practice of this invention is also useful as an aid in the clinical staging and prognosis of disease in AIDS or HIV-1 infected persons. As indicated, the skin test of this invention is not intended to be an immunizing dose. However, the administration of this test may result in an immunizing booster vaccination. It should be noted, however, that because of the long incubation period for AIDS or HIV-1, it is possible for unrecognized HIV-1 infection to be present at the time the test, the intradermal administration of the g160 protein, for example, is given.

In the practice of this invention the subcutaneous administration of the immunogenic AIDS virus envelope protein material should be avoided in HIV-1 seropositive persons. It is noted, however, that when tuberculosis is administered subcutaneously to test positive persons, no reaction occurs normally but a general febrile reaction and/or acute inflammation around old tuberculosis lesions may occur in highly sensitive individuals. It is cautioned, moreover, that seronegative persons may become seropositive for antibodies against AIDS or HIV-1 envelope proteins following the administration of the skin test in accordance with this invention.

### DETAILED DESCRIPTION OF THE INVENTION

The AIDS or HIV-1 virus infection test in accordance with this invention is carried out intradermally injecting with a syringe and needle 0.1 ml of gp160 solution and the test results are read by measuring the maximum extent of erythema and induration or by other tests, such as biopsy. In particular, the administration of the immunogenic AIDS virus envelope protein material in accordance with this invention is performed as follows:
1. The site of the test is usally the flexor or dorsal surface of the forearm about 10 cm (4 inch) below the elbow. Other skin sites may be used, but the flexor surface of the forearm is preferred.
2. The skin at the injection site is cleansed with 70% alcohol and allowed to dry.
3. The test material is administered with a tuberculin syringe (0.5 ml or 1.0 ml) fitted with a short [1.25 cm (1/2 inch)] 26 or 27 gauge needle.
4. The syringe and needle should be of a sterile disposable, single use type. A separate sterile unit should be used for each person tested.
5. The diaphragm of the vial stopper should be wiped with 70% alcohol.
6. The needle is inserted through the stopper diaphragm of the inverted vial. Exactly 0.1 ml is filled with into the syringe with care being taken to exclude air bubbles and to maintain the lumen of the needle filled.
7. The point of the needle is inserted into the most superficial layers of the skin with the needle bevel pointing upward. As the solution is injected, a pale bleb 6 to 10 mm size (approximately 1/4 inch to 1/2 inch) will rise over the point of the needle. This is quickly absorbed and no dressing is usually required.

In the event the injection is delivered subcutaneously (i.e. no bleb forms), or if a significant part of the dose leaks from the injection site, the test should be repeated immediately at another site at least 5 cm (2 inch) removed. The test protein materials may be stored at 2° - 8°C. for up to 3 months and as with any parenteral drug product, the test materials should be inspected visually for particulate matter and discoloration prior to administration.

It is important to use a separate sterile syringe and needle for each individual patient to prevent transmission of HIV, serum hepatitis virus and other infectious agents from one person to another. Where a syringe is employed, withdraw the recommended dose from the vial using a sterile needle and syringe free of preservatives, antiseptics and detergents.

Using standard techniques, readings of the DTH reaction should be made during the period from 48 to 72 hours after the injection and at any additional times prescribed. It is suggested that the boundaries marking the maximum extent of erythema and induration be delineated with a ballpoint pen, and then transferred to tape. One-half the sum of the perpendicular diameters should be used as the reaction size. Reactions greater than 5 mm (standard criteria) may be considered "positive". One diameter of the erythema and induration should be measured transversely to the long axis of the forearm. Additional tests, such as biopsy and histological examination for mononuclear cell infilration, may also be performed.

It should be noted that reactivity to the antigen material, e.g. gp160 may be non-specifically suppressed by non-HIV viral infections, live virus vaccines (i.e. measles, smallpox, polio, rubella and mumps), or by the administration of corticosteroids and malnutrition may also have a similar effect.

The immunogenic AIDS virus envelope protein material, such as gp160, is desirably supplied as a multi-dose vial in 2 ml containers with approximately 1.1 ml of antigen preparation per vial. Since the test materials contain no preservatives, the test materials should be used within 24 hours after opening the sealed containers. The sealed containers or vials are desirably stored at 2-8°C. and, as indicated, the unused contents should be discarded 24 hours after opening. Storage above or below the above-indicated recommended temperature may reduce potency and the antigen materials or solution should not be frozen since freezing destroys potency.

The immunogenic AIDS virus envelope protein test materials are desirably presented in kit form containing one or more 2.0 ml vials, together with a number of sterile syringes and needles adequate to take are of 0.1 ml doses from each 2 ml vial, such as 10 syringes and needles for each 2 ml vial of antigen solution.

As indicated hereinabove, it is preferred in the practices of this invention to employ gp160 antigen material, preferably gp160 antigen material expressed and derived from the AIDS virus envelope gene expressed through a baculorvirus in an insect cell, such as described in the aforementioned related U.S. patent application Serial No. 920,197. Other immunogenic fractions of the AIDS virus envelope protein material, in addition to gp160 or p24, usefully employed in the practices of this invention include the gp120 protein, gp40 protein and other immunogenic fractions thereof and of the envelope protein.

## Claims

1. Use of an immunogenic HIV envelope protein free of antigenic human proteins for preparing a sterile preservative-free aqueous solution for intradermal administration to a human to test for a delayed type hypersensitivity reaction to HIV antigen.

2. Use in accordance with claim 1 wherein the HIV envelope protein is genetically engineered or recombinant HIV envelope protein.

3. Use in accordance with claim 1 wherein said HIV envelope protein has been expressed from a recombinant insect virus.

4. Use in accordance with claim 1 wherein said HIV envelope protein has been expressed from a recombinant insect virus in an insect cell.

5. Use in accordance with claim 1 wherein said HIV envelope protein is gp160.

6. Use in accordance with claim 1 wherein said HIV envelope protein is gp120.

7. Use in accordance with claim 1 wherein said HIV envelope protein is expressed from a recombinant insect baculovirus.

8. Use in accordance with claim 1 wherein said HIV envelope protein is expressed from a recombinant insect baculovirus in an insect cell.

9. Use in accordance with claim 1 wherein said HIV envelope protein is gp40.

10. Use in accordance with claim 1 wherein said HIV envelope protein has been harvested and purified from cultures of Lepidopteran (insect) cells following exposure of the cells to a recombinant baculovirus vector containing HIV gene for HIV envelope protein.

11. Use in accordance with claim 10 wherein said gene is for the gp160 HIV envelope protein.

12. Use in accordance with claim 10 wherein said gene is for the gp120 HIV envelope protein.

13. Use in accordance with claim 1 wherein an amount of said HIV envelope protein sufficient for one intradermal injection is contained in a volume of about 0.1 ml of said aqueous solution.

14. Use of an immunogenic HIV envelope protein free of antigenic human proteins for preparing a test kit for intradermal administration for assessment of HIV specific cellular immunologic response in humans, said test kit comprising a sealed container containing said HIV envelope protein in a preservative-free aqueous solution, and at least one sterile disposable syringe and needle.

15. Use in accordance with claim 14 wherein the test kit additionally contains ethyl alcohol of at least a concentration of about 70% ethyl alcohol for cleansing the skin prior to the intradermal injection of the immunogenic HIV protein material.

## Patentansprüche

1. Verwendung eines immunogenen HIV-Hüllproteins, das frei von antigenen humanen Proteinen ist, zur Herstellung einer sterilen, von Konservierungsstoffen freien wäßrigen Lösung für die intradermale Verabreichung an einen Menschen, um auf eine Überempfindlichkeitsreaktion vom Spättyp gegen HIV-Antigen zu testen.

2. Verwendung nach Anspruch 1, bei der das HIV-Hüllprotein ein gentechnisch erzeugtes oder rekombinantes HIV-Hüllprotein ist.

3. Verwendung nach Anspruch 1, bei der das HIV-Hüllprotein von einem rekombinanten Insektenvirus exprimiert wurde.

4. Verwendung nach Anspruch 1, bei der das HIV-Hüllprotein von einem rekombinanten Insektenvirus in einer Insektenzelle exprimiert wurde.

5. Verwendung nach Anspruch 1, bei der das HIV-Hüllprotein gp160 ist.

6. Verwendung nach Anspruch 1, bei der das HIV-Hüllprotein gp120 ist.

7. Verwendung nach Anspruch 1, bei der das HIV-Hüllprotein von einem rekombinanten Insekten-Baculovirus exprimiert wurde.

8. Verwendung nach Anspruch 1, bei der das HIV-Hüllprotein von einem rekombinanten Insekten-Baculovirus in einer Insektenzelle exprimiert wurde.

9. Verwendung nach Anspruch 1, bei der das HIV-Hüllprotein gp40 ist.

10. Verwendung nach Anspruch 1, bei der das HIV-Hüllprotein aus Kulturen von Lepidoptera- (Insekten-) Zellen geerntet und gereinigt wurde, nachdem die Zellen einem rekombinanten Baculovirus-Vektor ausgesetzt worden waren, der das HIV-Gen für das HIV-Hüllprotein enthielt.

11. Verwendung nach Anspruch 10, bei der das Gen das für das gp160 HIV-Hüllprotein ist.

12. Verwendung nach Anspruch 10, bei der das Gen das für das gp120 HIV-Hüllprotein ist.

13. Verwendung nach Anspruch 1, bei der eine Menge des genannten HIV-Hüllproteins, die für eine intradermale Injektion ausreicht, in einem Volumen von etwa 0,1 ml der genannten wäßrigen Lösung enthalten ist.

14. Verwendung eines immunogenen HIV-Hüllproteins, das frei von antigenen humanen Proteinen ist, zur Herstellung eines Testkits für die intradermale Verabreichung zur Bestimmung einer HIV-spezifischen zellulären immunologischen Reaktion bei Menschen, wobei der Testkit einen verschlossenen Behälter, der das genannte HIV-Hüllprotein in einer von Konservierungsstoffen freien wäßrigen Lösung enthält, sowie wenigstens eine sterile Einwegspritze und -nadel aufweist.

15. Verwendung nach Anspruch 14, bei der der Testkit zusätzlich Ethylalkohol mit einer Konzentration von wenigstens etwa 70% Ethylalkohol zum Reinigen der Haut vor der intradermalen Injektion des immunogenen HIV-Proteinmaterials enthält.

## Revendications

1. Utilisation d'une protéine immunogénique de l'enveloppe de HIV exempte de protéines antigéniques humaines pour préparer une solution aqueuse stérile exempte de conservateurs pour une administration intradermique à un être humain pour tester une réaction d'hypersensibilité de type retardé à un antigène de HIV.

2. Utilisation conformément à la revendication 1 dans laquelle la protéine de l'enveloppe de HIV est une protéine de l'enveloppe de HIV recombinante ou génétiquement modifiée.

3. Utilisation conformément à la revendication 1 dans laquelle ladite protéine de l'enveloppe de HIV a été exprimée à partir d'un virus d'insecte recombinant.

4. Utilisation conformément à la revendication 1 dans laquelle ladite protéine de l'enveloppe de HIV a été exprimée à partir d'un virus d'insecte recombinant dans une cellule d'insecte.

5. Utilisation conformément à la revendication 1 dans laquelle ladite protéine de l'enveloppe de HIV est gp160.

6. Utilisation conformément à la revendication 1 dans laquelle ladite protéine de l'enveloppe de HIV est gp120.

7. Utilisation conformément à la revendication 1 dans laquelle ladite protéine de l'enveloppe de HIV est exprimée à partir d'un baculovirus d'insecte recombinant.

8. Utilisation conformément à la revendication 1 dans laquelle ladite protéine de l'enveloppe HIV est exprimée à partir d'un baculovirus d'insecte recombinant dans une cellule d'insecte.

9. Utilisation conformément à la revendication 1 dans laquelle ladite protéine de l'enveloppe de HIV est gp40.

10. Utilisation conformément à la revendication 1 dans laquelle ladite protéine de l'enveloppe de HIV a été récoltée et purifiée à partir de cultures de cellules de Lepidoptère (insecte) après l'exposition des cellules à un vecteur de baculovirus recombinant contenant le gène de HIV pour la protéine de l'enveloppe de HIV.

11. Utilisation conformément à la revendication 10 dans laquelle ledit gène est pour la protéine de l'enveloppe de HIV gp160.

12. Utilisation conformément à la revendication 10 dans laquelle le gêne est pour la protéine de l'enveloppe de HIV gp120.

13. Utilisation conformément à la revendication 1 dans laquelle une quantité de ladite protéine de l'enveloppe de HIV suffisante pour une injection intradermique est contenue dans un volume d'environ 0,1 ml de ladite solution aqueuse.

14. Utilisation d'une protéine immunogénique de l'enveloppe de HIV exempte de protéines humaines antigéniques pour préparer un kit test pour une administration intradermique pour l'évaluation de la réponse immunologique cellulaire spécifique à HIV chez les êtres humains, ledit kit de test comprenant un conteneur scellé contenant ladite protéine de l'enveloppe de HIV dans une solution aqueuse exempte de conservateurs, et au moins une seringue et une aiguille jetables stériles.

15. Utilisation conformément à la revendication 14 dans laquelle le kit de test contient de plus de l'alcool éthylique à au moins une concentration d'environ 70% d'alcool éthylique pour nettoyer la peau avant l'injection intradermique de la matière protéique immunogénique de HIV.
